# EUROPEAN PATENT APPLICATION

(11) **EP 3 543 698 A1**
(43) Date of publication of application: **25.09.2019**
(21) Application number: 18162800.9
(22) Date of filing: 20.03.2018
(51) Int. Cl.: G01N 33/569, A61K 39/00, G01N 33/68

(54) **METHOD FOR DETERMINING VACCINE EFFICACY IN AN INDIVIDUAL AND MEANS THEREFORE**

(71) Applicant: Helmholtz-Zentrum für Infektionsforschung GmbH, 38124 Braunschweig (DE)
(72) Inventor: Pessler, Frank, 30519 Hannover (DE); Guzman, Carlos, 38304 Wolfenbüttel (DE); Riese, Peggy, 38110 Braunschweig (DE); Akmatov, Manas, 38124 Braunschweig (DE)
(74) Representative: Gramm, Lins & Partner Patent- und Rechtsanwälte PartGmbB

(57) **Abstract**

In a first aspect, the present invention relates to a method for determining the responsiveness of an individual to vaccination, like viral vaccination or for the determination of viral vaccine efficacy in an individual as well as a method for the stratification of the vaccination regimen, e.g. viral vaccination, in an individual based on determining the level or the amount of at least one of IL-8 or IL-18 in a sample; said sample is obtained from an individual at least once before or at least once after vaccination. The method allows to determine the vaccination regimen with the vaccine, in particular, a virus vaccine, like an influenza virus vaccine whereby when a low level of at least one of IL-8 and/or IL-18 is determined, said low level is indicative for a personalized vaccine strategy. In a further aspect, the use of at least one of IL-8 or IL-18 as a predictive marker for vaccine efficacy or immune protection by vaccination is provided. Finally, a kit of parts for vaccination comprising equipment for determining the level and/or amount of at least one of IL-8 or IL-18 in a sample obtained from an individual to be vaccinated as well as the vaccine to be administered is described.

## Description

In a first aspect, the present invention relates to a method for determining the responsiveness of an individual to vaccination, like viral vaccination or for the determination of vaccine efficacy, like viral vaccine efficacy in an individual as well as a method for the stratification of the vaccination regimen, e.g. viral vaccination, in an individual based on determining the level or the amount of at least one of IL-8 or IL-18 in a sample; said sample is obtained from an individual at least once before or at least once after vaccination. The method allows to determine the vaccination regimen with the vaccine, in particular, a virus vaccine, like an influenza virus vaccine whereby when a low level of at least one of IL-8 and/or IL-18 is determined, said low level is indicative for a personalized vaccine strategy. In a further aspect, the use of at least one of IL-8 or IL-18 as a predictive marker for vaccine efficacy or immune protection by vaccination is provided. Finally, a kit of parts for vaccination comprising equipment for determining the level and/or amount of at least one of IL-8 or IL-18 in a sample obtained from an individual to be vaccinated as well as the vaccine to be administered is described.

### Prior art

Determining the efficacy of vaccination is important to allow protective vaccination of individuals. However, there is always a minor group of individuals where vaccination is ineffective. For example, influenza infection carries a high morbidity and mortality in particular among elderly individuals. The same is true for individuals with chronic lung diseases such as chronic obstructive pulmonary disease (COPD). On the other hand, the incidence rate by influenza infection is highest among elderly individuals (>60 years). In addition, elderly individuals have a higher risk of severe influenza infection, and the risk of death due to influenza infection is highest as well. Seasonal influenza vaccination represents one of the most effective preventive measures for influenza-associated complications. However, it was demonstrated in the art that the immunological protective immune response of the influenza vaccine as well as the efficacy of the vaccination decrease with increasing age.

In 1999 it was estimated that the total annual associated cost of influenza infection in Germany was approximately 888 million Euros and a 2016 study estimated the direct cost of influenza-associated health care to be approximately 100 million Euros. Depending on the influenza season, globally between 250.000 and 500.000 people die from an influenza infection per year in recent years. In Germany, the estimated number of influenza related deaths is between 5.000 and 15.000. Of note, most deaths, namely, over 90%, occur in the age group of >60 years. Hence, influenza vaccination is recommended for individuals over 60 years and in Germany about 8 Million individuals in this age group receive the vaccine annually.

However, depending on the vaccine strain, up to 80% of vaccinees in this age group do not develop protective titers, and presumably more immunogenic adjuvant flu vaccines, e.g. Fluad®, Seqirus, are being developed. However, also these newly developed vaccines still have the problem that a considerable number of individuals do not respond thereto. Even the newly developed vaccines based on recombinant antigens and not on attenuated vaccines fail to increase the responsiveness. For example, Fluad® contains three different antigens and a full response to all these antigens is observed only in a minority of vaccinees.

Individuals with COPD have an increased risk of complicated influenza infection. A recent study suggested that the response to influenza vaccination in COPD patients is severely reduced. Hence, if all 7 million individuals in Germany with COPD received, as recommended, the seasonal influenza vaccine, 3 to 4 million doses of said vaccine would be administered without effect. However, there are no predictive markers or parameters available that would be useful for identifying individuals poorly responding or non-responding to vaccination. That is, there is no predictive test for poor or non-immune response available. Such test would allow identifying individuals in need of specialized vaccination strategies apart from the "normal" vaccine strategy applied so far. The specialized vaccination strategy or personalized vaccination strategy allows to increase responsiveness to the vaccination, thus, increasing the number of individuals having a sufficient immune response against viral vaccination, in particular, influenza vaccination.

Van Reeth K., et al, Veterinary Microbiology, 2000, 74, 109-116, describes cytokines in the pathogenesis of influenza. Further, Van Reeth, K., et al, Viral Immunology, 2002, 15 (4), 583-594, describe a correlation between lung proinflammatory cytokine levels, virus replication, and disease after swine influenza virus challenge of vaccination-immune pigs.

Zhang, X., et al, Research in Veterinary Science, 2013, 94, 346-353, identifies a positive inductive effect of IL-18 on virus specific immune responses induced by PRRSV-GP5 DNA vaccine in swine. Therein, it is considered that IL-18 has a positive inductive effect on the activation of cellular immune responses in swine. Further, animal studies have shown that IL-18 can enhance vaccine responses in mice and pigs.

However, there is a need in humans for improving vaccination efficacy and personalized vaccination strategies for example using higher doses, repeated injection or adjuvant vaccines, thus, increasing the percentage of immune protective vaccinated individuals.

### Brief description of the present invention

In a first aspect, the present invention provides a method for determining the responsiveness of an individual to vaccination or for the determination of vaccine efficacy in an individual comprising the step of
determining the level and/or the amount of at least one of IL-8 or IL-18 in a sample obtained from said individual whereby said sample is obtained at least once before or at least once after vaccination;
comparing the level and/or amount of at least one of IL-8 or IL-18 in said sample with a reference level and/or reference amount;
wherein a low level or decreased level of said at least one of IL-8 or IL-18 is indicative for a low responsiveness of said individual to vaccination, or for a low vaccine efficacy in said individual.

In a further aspect, the present invention relates to a method for the stratification of the viral vaccination regimen in an individual comprising the step of determining the level and/or amount of at least one of IL-8 or IL-18 in a sample obtained from said individual, said sample is obtained at least once before and/or at least once after at least the first vaccination;
determining the vaccination strategy based on the level and/or amount of at least one of IL-8 or IL-18 in said sample.

The above methods are particularly useful for viral vaccination and with viral vaccine.

Further, the present invention provides a method for determining the vaccination regimen with a vaccine, like a virus vaccine, in particular, an influenza virus vaccine, comprising the step of
determining the level and/or amount of at least one of IL-8 or IL-18 in a sample obtained from the individual to be vaccinated;
wherein a low level of at least one of IL-8 and/or IL-18 is indicative for a personalized vaccine strategy wherein at least one of a higher dosage, a repeated injection, or an adjuvanted vaccine, or a vaccine otherwise modified to enhance the vaccine response is to be administered.

Moreover, the present inventors aim to identify IL-8 or IL-18 as a predictive marker useful for determine vaccine efficacy or immune protection by vaccination, like virus vaccination.

Furher. the present invention provides a kit of parts for vaccination including virus vaccination comprising equipment for determining the level and/or amount of at least one of IL-8 or IL-18 in a sample obtained from the individual to be vaccinated and the vaccine including the virus vaccine to be administered after determining the amount or level of at least one of IL-8 or IL-18 in said sample.

Finally, the present invention relates to the use of at least one of IL-8 or IL-18 protein in a vaccine, in particular, a viral vaccine, like influenza vaccine, for increasing immune protection or for increasing vaccine efficacy in an individual to be vaccinated.

### Brief description of the drawings

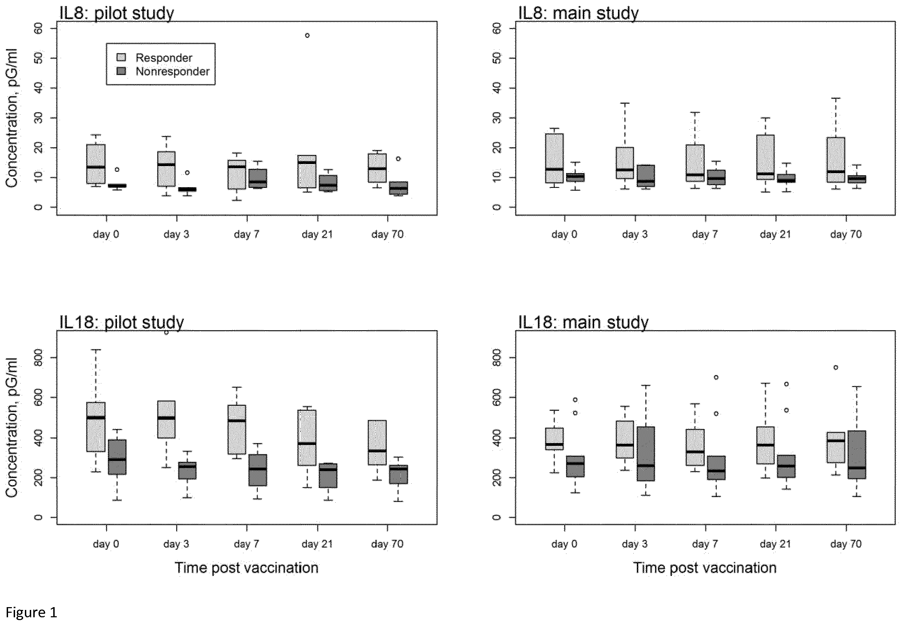

Fig. 1. Microbead (Luminex) measurements illustrating low levels of IL-8 (top row) and IL-18 (bottom row) in a pilot study (left two panels; n=32 participants) and a main study (right two panels; n=200 participants) on the immune response to influenza vaccination in individuals ≥65 years of age. A non-response was defined as lack of a 4-fold increase in titers to all three HA-antigens (H1N1, H3N2, B) contained in the vaccine (Fluad™).

### Detailed description of the present invention

In a first aspect, the present invention relates to determining the responsiveness of an individual to vaccination or for the determination of viral vaccine efficacy in an individual comprising the step of
determining the level and/or the amount of at least one of IL-8 or IL-18 in a sample obtained from said individual whereby said sample is obtained at least once before or at least once after vaccination;
comparing the level and/or amount of at least one of IL-8 or IL-18 in said sample with a reference level and/or reference amount;
wherein a low level or decreased level of said at least one of IL-8 or IL-18 is indicative for a low responsiveness of said individual to vaccination, or for a low vaccine efficacy in said individual.

In an alternative embodiment of the present invention, the present invention relates to a method for the stratification of the vaccination regimen, like the viral vaccination regimen in an individual comprising the step of determining the level and/or amount of at least one of IL-8 or IL-18 in a sample obtained from said individual, said sample is obtained at least once before or at least once after at least the first vaccination; determining the vaccination strategy based on the level and/or amount of at least one of IL-8 or IL-18 in said sample.

Further, another aspect of the present invention relates to a method for determining the vaccination regimen with a vaccine, like a virus vaccine, in particular, an influenza virus vaccine, comprising the step of
determining the level and/or amount of at least one of IL-8 or IL-18 in a sample obtained from the individual to be vaccinated;
wherein a low level of at least one of IL-8 and/or IL-18 is indicative for a personalized vaccine strategy wherein at least one of a higher dosage, a repeated injection, or an adjuvanted vaccine, or a vaccine otherwise modified to enhance the vaccine response is to be administered.

In an embodiment of the present invention, the sample from said individual is obtained at least once before or at least once after at least the first vaccination.

The term "comprising" as used herein is open ended and means that the subject matter must contain all the features specifically recited therein, but there is no bar of additional features that are not recited being present as well. The term "comprising" or "comprise" is used interchangeably herein with the term "contain". A specific embodiment of "comprising" or "containing" is "consisting of".

Several documents are cited throughout the text of the specification. Each of the documents cited herein (including all patents, patent applications, scientific publications, manufacturers specifications, instructions, etc.) whether supra or infra, are hereby incorporated by reference in their entirety. Nothing herein is to be construed as an ambition that the invention is not entitled to anti-date such disclosure by virtue of prior invention.

As used herein, the terms "subject", "individual" and "object" are used herein synonymously. The term "subject", "individual" and "object" means warm blooded mammals such as humans and primates as well as farm and other domestic animals, laboratory animals, such as mice, rats and guinea pigs and zoo and wild animals and the like as well as fish for consumption, e.g., reared in aquaculture.

The term "determining" as used herein refers to assessing the presence, absence, quantity, level or amount of either a given substance within a clinical or individual derived sample, including qualitative or quantitative concentration levels of substances, or otherwise evaluating the values or categorizing of an individual's parameter.

As used herein, the terms "treatment" or "treating" refer to both a therapeutic treatment and prophylactic or preventative measures unless otherwise identified.

The similar terms "a", "an" and "the" include plural reference unless the context clearly indicates otherwise.

Unless otherwise indicated, the terms "IL-8" and "IL-18" include the protein and the nucleic acid molecule encoding the same.

The present inventors recognized that at least one of IL-8 or IL-18 represents a predictive biomarker for a poor response to viral vaccination and, thus, represents a suitable marker to determine the susceptibility of an individual to viral vaccination before administering said vaccine. In the following, the terms "susceptibility" and "responsiveness" will be used interchangeably unless otherwise indicated.

That is, the present inventors recognized that when the level of at least one of IL-8 or IL-18 is low or decreased compared to a reference level and/or a reference amount, both of them are also referred to the term "reference value" the susceptibility of an individual to viral vaccination is low as well as the efficacy. Further, a personalized vaccine strategy is required. In the context of the present invention, the term "reference value" refers to an index value, a value derived from one or more computer indices, a value derived from an individual or a cohort of individuals being successfully vaccinated and, in addition, the reference value represents a range or index obtained from at least two samples collected from individuals accordingly.

Further, determining the level and/or amount of at least one of IL-8 or IL-18 as a predictive biomarker allows for the stratification of the vaccination regimen, like the viral vaccination regimen. That is, personalization of the vaccination regimen, like the viral vaccination regimen in an individual is possible. The stratification requires determining at least one of IL-8 or IL-18 in said sample either at least once before and/or once after the first vaccination and determining the vaccination regimen based on the level and/or amount of the at least one IL-8 or IL-18 of said individual. For example, in case of low or decreased levels of at least one of IL-8 or IL-18, the doses have to be increased. Alternatively or in addition, a repeated injection may be required. Further, the regimen may include the use of specific types of vaccines, e.g. adjuvant vaccines, for increasing the percentage of immune protective vaccinated individuals.

That is, the present inventors noted that in case of low levels or amounts of at least one of IL-8 or IL-18 in said individual, a personalized or adapted vaccination strategy is required to achieve vaccine efficacy in said individual. Hence, the high rate of non-responders in particular in the group of elderly persons can be decreased accordingly. Hence, a personalization is possible.

In the method for determining the vaccination regimen with a vaccine, like a virus vaccine, in particular an influenza virus vaccine according to the present invention, the method comprises determination of the level or amount of the at least one of IL-8 or IL-18 in a sample obtained from the individual to be vaccinated and when a low level or amount of at least one of IL-8 and IL-18 is determined, the low level or low amount is indicative for a personalized vaccine strategy, e.g. wherein at least one of a higher dosage, a repeated injection or an adjuvant vaccine, or a vaccine otherwise modified to enhance vaccine response, is to be administered.

The vaccination and the vaccine may be one known in the art. E.g. the vaccine may be a vaccine against bacteria, virus or parasites, in particular, virus. Further, the vaccine may be a subunit vaccine like a protein-based vaccine or of a whole pathogen, like bacteria, parasites or virus.

In an embodiment of the present invention, the vaccination is a viral vaccination e.g. against a virus like an RNA, preferably of the family of Orthomyxoviridae in particular, an influenza virus. But application in the context of vaccines against DNA viruses or even bacteria is conceivable.

In an embodiment, the viral vaccination is a vaccination against the influenza virus including influenza virus A, influenza virus B and influenza virus C.

The vaccine may include attenuated virus and/or a mixture of isolated viral proteins. That is, viral vaccines known in the art may be applied including the influenza virus vaccines commercially available like Afluria, Begripal, Fluad®, and several others including Fluenz Tetra, Influsplit Tetra, Nfluvac, Vaxigrip, Xanaflu, Fluarix, IDflu, IN-TANZA, Optaflu.

In an embodiment of the method according to the present invention the virus against which the vaccination should be used is an influenza virus and the virus vaccination is a vaccination with recombinant HA-proteins of said influenza virus comprising at least one HA-protein from influenza A and at least one HA-protein from influenza B.

In particular, with respect to the vaccination campaign for seasonal vaccination against e.g. Influenza, the method according to the present invention may be applied using the predictive biomarker disclosed herein, namely at least one of IL-8 or IL-18 for determining the responsiveness of an individual against said vaccination, like viral vaccination. Thus, it is possible to determine the vaccination regimen and, if necessary, personalize the vaccine strategy to allow efficient and immunoprotective vaccination accordingly.

The sample obtained from said individual is in one embodiment, a blood sample including a plasma sample or serum sample. The sample may also be obtained from other body fluid or tissue, like sputum. In a specific embodiment, the sample is a blood sample, in particular, a serum sample.

Determination of the level or amount of at least one of IL-8 or IL-18 may be conducted by known methods. In an embodiment, the level or amount of the at least one of IL-8 or IL-18 is determined on protein level. For example, determination is effected by immunoassays including ELISpot, ELISA, lateral slide test, microbeads based systems, e.g. known from Luminex, microfluidics based device.

For example, the level or amount of the at least one of IL-8 or IL-18 is determined in the sample of the individual before seasonal vaccination.

In particular, the method is conducted when considering to use an adjuvanted virus vaccine for vaccination.

The methods according to the present invention are particularly useful when the individual is a human, in particular, an elderly human of an age of 60 years or above, like 65 years or above.

As discussed, the frequency of non-responders to virus vaccination in this cohort is high, on the other hand, the seasonal influenza vaccination is recommended specifically for individuals of an age of 60 years or above. Further, the mortality in this cohort of elderly human of an age of 60 years or above is high. Hence, the methods according to the present invention are particularly useful for individuals being elderly human of an age of 60 years or above, like 65 years or above.

Moreover, the methods according to the present invention are useful for individuals suffering from COPD since also in this cohort of individuals, the mortality rate after virus infection, like influenza infection, is higher. It was demonstrated before that influenza infection is one of the frequently occurring causes of COPD exercabation. Hence, patients suffering from COPD are recommended for seasonal influenza vaccination. However, also in this group of individuals, a lowered immune response after vaccination occurs. For example, the seroconversion rate of healthy individuals is about 90% while only about 43% in patients suffering from COPD, Nath et al., 2014, Int J Chron Obstruct Pulmon Dis; 9:51-6.

Hence, the methods according to the present invention are also useful for COPD individuals.

In addition, the individuals are individuals suffering from metabolic dysfunction including individuals having altered BMI or affected by diabetes.

In an embodiment of the present invention the method according to the present invention comprises further the individual administration of the viral vaccine by the physician. In particular, based on determining the level or amount of at least one of IL-8 or IL-18, the physician personalizes the vaccine strategy, for example by selecting a specific vaccine, e.g. adjuvanted vaccine, increasing the dosage or repeating administration of vaccine, or use of highly sophisticated "next generation" vaccines.

In another embodiment the present invention will also be useful in decision-making regarding inclusion/exclusion of eligible individuals in clinical trials on vaccines, in particular studies on presumably more effective vaccines and/or vaccination strategies. For instance, low plasma levels of IL-8 and/or IL-18 could be used as an inclusion criterion in order to enrich the study population in individuals at higher risk of a vaccine non-response.

Hence, in a further aspect, the present invention relates to a method of vaccination of individuals against virus, in particular, influenza virus. Said vaccination may be prophylactic or therapeutic vaccination.

The method of vaccination of the individual includes the method of determining susceptibility or the method of stratification or determining the treatment course as well as determining the efficacy of vaccination in the individual to be vaccinated and, depending on the level or amount of at least one of IL-8 or IL-18, administering the selected vaccine as described above. That is, when determining low levels or amount of at least one of IL-8 or IL-18 in said individual, a personalized vaccination should be conducted accordingly.

In a further aspect, the method for vaccination includes the use of at least one of IL-8 or IL-18 protein or nucleic acid molecule (e.g. DNA or RNA) encoding the same in a vaccine, in particular, a viral vaccine, like influenza vaccine for increasing immune protection or for increasing vaccine efficacy in an individual to be vaccinated. The administration of at least one of IL-8 or IL-18 may be conducted simultaneously, separately, or sequentially. The skilled person is well aware of suitable ways of administration of the combination of vaccine with at least one of the IL-8 or IL-18 according to the present invention. The present inventors recognized that increasing the level or amount of IL-8 or IL-18 in the individual to be vaccinated increase the change to obtain immunoprotective immune response in said individual and increasing the vaccine efficacy. In an embodiment of the present invention, the at least one of IL-8 or IL-18 is administered in advance and the vaccine is administered at a later time point. The skilled person can easily determine suitable time points of administration.

The route of administration is selected by the skilled person accordingly. For example, the vaccination may occur subcutaneously or intramuscularly, and administration of at least one of IL-8 or IL-18 may also be by way of subcutaneous or intramuscular injection.

Moreover, the present invention relates in a further aspect to the use of at least one of IL-8 or IL-18 as a predictive marker for vaccine efficacy or immune protection by vaccination, like virus vaccination. The usefulness of said predictive marker is demonstrated herein. This is particularly true for determining the efficacy of vaccination using an adjuvanted vaccine or a vaccine containing recombinant or purified natural viral proteins, like Fluad®.

Further, the present invention describes the use of at least one of IL-8 and/or IL-18 for determining the vaccination strategy with a vaccine, like a viral vaccine, in particular, an influenza vaccine.

In a further aspect, the present invention relates to a kit of parts for vaccination, like virus vaccination. This kit of parts comprises at least the necessary equipment for determining the level and/or the amount of at least one of IL-8 or IL-18 in a sample obtained from the individual to be vaccinated and the vaccine, like the viral vaccine to be administered to said individual after the level and/or amount of the at least one of IL-8 or IL-18 is determined.

For example, the necessary equipment is an ELISA assay or an ELISpot assay or any other immuno assay for determining the level and/or amount of at least one of IL-8 and/or IL-18. Alternatively, the equipment is a chip system or any other system allowing determination of the level or amount of at least one of IL-8 or IL-18 accordingly. In case of determining the level or amount of IL-8 or IL-18 on nucleic acid level, the skilled person is well aware of suitable methods and equipment including equipment required for PCR techniques, etc. For example, the equipment is a test allowing rapid determination of the level or amount of at least one of IL-8 or IL-18, thus, allowing the physician to determine the vaccine strategy accordingly.

The kit of parts according to the present invention includes further the vaccine to be administered. For example, the vaccine may be provided in different doses or in more than one doses and the skilled person can determine the number of doses to be administered based on the level or amount of at least one of IL-8 and IL-18 determined before.

In an embodiment of the present invention, the kit of parts comprises an equipment being selected from ELISA if at least one of IL-8 or IL-18 is determined on the protein level or, alternatively, a lateral slide test or a microfluidic spaced device.

In an embodiment of the kit of parts according to the present invention, the vaccine, like the virus vaccine being part of said kit of parts is an attenuated virus vaccine or is a virus vaccine containing recombinant or purified single viral components, in particular, HA-proteins.

The vaccine, like the viral vaccine is provided in a suitable form accordingly.

The present invention will be described further by way of examples without limiting the same thereto.

### Examples

### Methods

The data described herein were obtained in the context of two independent studies on influenza vaccination of individuals ≥65 of age.

### 1) Pilot study: study design and population

The pilot study was a prospective population-based study spanning the time period between December 2014 and May 2015. In brief, a random sample of individuals between 65 and 80 years of age (N=1429) was drawn from the residents' registration office in Hannover, Germany. The individuals received an invitation letter by mail to participate in the study. Those who agree to participate were invited to the Clinical Research Center (CRC) Hannover. In the study center they underwent medical examinations, collection of data through self-administered questionnaire, collection of blood samples (∼50 ml) and subsequent influenza vaccination with an inactivated, trivalent, adjuvanted (MF59) vaccine against seasonal influenza (Fluad™, Novartis Vaccines and Diagnostics S.r.l., Rosia, Italy) (day 0). Further blood samples were taken 3, 7, 21 and 70 days after vaccination. In total, 34 study participants were recruited.

### 2) Main study: study design and population

The sampling of the study population and study design were identical to those of the pilot study. The study was conducted in the next year (from September 2015 to May 2016). Some changes were adapted, including a) increasing the number of initial invitations (N=5582) to ensure a sufficient sample size, b) oversampling the older age group (76-80 years) to ensure an adequate sample size in this age group, c) conduct of a nonresponder survey to examine a potential nonresponse bias, and d) collection of a blood sample on day 1 from half of the participants and on day 3 from the other half. The final sample size was 200 participants. Further details of the applied methodology in the pilot and main studies can be found elsewhere [Akmatov MK, et al., Hum Vaccin Immunother. 2017;10:10, Akmatov MK, , et al. BMC Med Res Methodol. 2017;17:18.]

Determination of responders and nonresponders to influenza vaccination A hemagglutination inhibition (HAI) assay was used to distinguish between vaccine responders and nonresponders. At least four-fold titer increase in blood samples of day 0 (before vaccination) and day 21 post-vaccination was considered a responder for the respective antigen (i.e. H1 N1, H3N2 and B). We defined total responders as those who had at least four-fold titer increase in all three antigens. Correspondently, total nonresponders were those who did not have a four-fold titer increase in all three antigens.

### Cytokine profiling

Cytokine profiles were determined using a microbead-based assay (Luminex). A total of 52 targets were thus measured in plasma aliquots from the study participants. 38 of these passed quality assessment (see below) and were included in statistical analyses.

### Statistical analysis

All analyses were done separately for the pilot and main studies. Initially, we performed missing data analysis. Of the total 51 cytokines, 32 (63%) and 31 (61 %) had no missing values at all in the pilot and main study, respectively. In 15 (29%) and 13 (25%) cytokines the proportion of missing values was more than 30% in the pilot and main study, respectively. Of them, 13 cytokines (same in both studies; ET-1, GM-CSF, IL-1alpha, IL-2, IL-3, IL-5, IL-7, IL-12 p70, IL-13, IL-15, IL-25, lymphotactin, and TNF-beta) were excluded from further analyses. For the remaining 38 cytokines we performed the Little's chi-square statistic to test whether values were missing completely at random (i.e. MCAR), Little RJA. J American Statistical Association. 1988;83. Little's test revealed χ=688.329, df=675, p=0.353 for the pilot study and χ=420.554, df=401, p=0.241 for the main study. We thus included all 38 cytokines in further analyses. As a next step, classical multidimensional scaling (MDS) was used to examine the level of (dis)similarity of responders and nonresponders at different time points by cytokines. Furthermore, Receiver Operating Characteristic (ROC) curve analysis was used to assess the discriminatory ability of cytokines to differentiate between total responders and total nonresponders. ROC curve analysis was performed by applying binary ROC curve analysis separately for each time point. The dependent variable in binary ROC curve analyses was the response to influenza vaccination, i.e. total nonresponders vs. total responders. Due to the higher "n" in the main study, we were able to select sex and age matches for the total nonresponders from the pool of total responders. The independent variables in binary ROC curve analyses were cytokines measured at each time point (n=190).

The analyses were done with IBM SPSS Statistics for Windows, version 19 (IBM Corporation, Armonk, NY, USA) and the R Foundation for Statistical Computing, version 3.3.2, R: language and environment for statistical computing. www.r-project.org.

| **Table 1. Diagnostic accuracy of IL-8 and IL-18 plasma concentrations to predict a non-response to influenza vaccination in individuals ≥65 y.** | | |
|---|---|---|
| **Cytokine** | **AUC (95% CI)** | |
| | **Pilot** | **Main** |
| IL8 day 0 | 0.89 (0.70-1.00) | 0.68 (0.43-0.93) |
| IL8 day 3 | 0.78 (0.49-1.00) | 0.70 (0.46-0.93) |
| IL8 day 7 | 0.58 (0.21-0.96) | 0.64 (0.38-0.89) |
| IL8 day 21 | 0.75 (0.43-1.00) | 0.70 (0.45-0.94) |
| IL8 day 70 | 0.86 (0.65-1.00) | 0.69 (0.44-0.94) |
| IL18 day 0 | 0.81 (0.55-1.00) | 0.74 (0.49-0.98) |
| IL18 day 3 | 0.90 (0.70-1.00) | 0.70 (0.45-0.94) |
| IL18 day 7 | 0.92 (0.73-1.00) | 0.75 (0.51-0.99) |
| IL18 day 21 | 0.79 (0.49-1.00) | 0.70 (0.46-0.95) |
| IL18 day 70 | 0.83 (0.59-1.00) | 0.65 (0.38-0.91) |
| The analysis is based on the data presented in Fig. 1. The item "day" refers to the time before (day 0) | | |
| or after (day 3-70) vaccination. AUC = are under the receiver operating characteristic (ROC) curve | | |
| (AUC of 1 = perfect discrimination, AUC of 0.5 = no discrimination). CI = confidence interval. | | |

## Claims

1. A method for determining the responsiveness of an individual to viral vaccination or for the determination of vaccine efficacy in an individual comprising the step of
determining the level and/or the amount of at least one of IL-8 or IL-18 in a sample obtained from said individual whereby said sample is obtained at least once before or at least once after vaccination;
comparing the level and/or amount of at least one of IL-8 or IL-18 in said sample with a reference level and/or reference amount;
wherein a low level or amount or decreased level or amount of said at least one of IL-8 or IL-18 is indicative for a low responsiveness of said individual to vaccination, or for a low vaccine efficacy in said individual.

2. A method for the stratification of the viral vaccination regimen in an individual comprising the step of determining the level and/or amount of at least one of IL-8 or IL-18 in a sample obtained from said individual, said sample is obtained at least once before and/or at least once after at least the first vaccination; determining the vaccination regimen based on the level and/or amount of at least one of IL-8 or IL-18 in said sample.

3. A method for determining the vaccination regimen with a vaccine, like virus vaccine, in particular, an influenza virus vaccine,
comprising the step of
determining the level and/or amount of at least one of IL-8 or IL-18 in a sample obtained from the individual to be vaccinated;
wherein a low level or amount of at least one of IL-8 and/or IL-18 is indicative for a personalized vaccine strategy wherein at least one of a higher dosage, a repeated injection, or an adjuvanted vaccine, or a vaccine otherwise modified to enhance the vaccine response is to be administered.

4. A method according to claim 1 for stratifying participants in vaccine trials, in particular, to enrich a study population in individuals at high risk of a vaccine non-response.

5. The method according to any one of the preceding claims wherein the vaccination is a viral vaccination, in particular, a vaccination is against a virus, like a RNA virus, preferably of the family of Orthomyxoviridae, in particular an influenza virus.

6. A method according to any one of the preceding claims wherein the vaccination is a viral vaccination and the virus present in the viral vaccine is an attenuated virus or is a mixture of isolated viral proteins.

7. The method according to any one of the preceding claims wherein the virus against the vaccination is directed is an influenza virus and the virus vaccination is a vaccination with recombinant HA-proteins of the influenza virus comprising at least one HA-protein from influenza A and at least one HA-protein from influenza B.

8. The method according to any one of the preceding claims wherein the sample is a blood sample including a plasma sample or serum sample.

9. The method according to any one of the preceding claims wherein the determination of at least one of IL-8 or IL-18 is on protein level.

10. The method according to any one of the preceding claims wherein the vaccine, like the virus vaccine, is an adjuvanted vaccine.

11. The method according to any one of the preceding claims wherein the individual is a human, in particular, an elderly human of an age of 60 years or above, like 65 years or above.

12. The use of at least one of IL-8 or IL-18 i) as a predictive marker for vaccine efficacy or immune protection by vaccination or ii) for determining the vaccination strategy with a vaccine, in particular, a virus vaccination and a viral vaccine, an influenza vaccine.

13. A kit of parts for virus vaccination comprising equipment for determining the level and/or amount of at least one of IL-8 or IL-18 in a sample obtained from the individual to be vaccinated, and the vaccine to be administered, in particular, wherein the equipment for determining the level or amount of at least one of IL-8 or IL-18 is an assay for determining the level and/or amount of IL-8 or IL-18 on protein level, in particular, an ELISA, or a lateral slide test or a microfluidics based device.

14. A kit of parts according to claim 13 wherein the vaccine is a virus vaccine in particular is an attenuated virus vaccine or is a virus vaccine containing recombinant or isolated single viral components, in particular, HA proteins.

15. At least one of IL-8 or IL-18 protein or nucleic acid encoding the same for use in a vaccine, in particular, a viral vaccine, like influenza vaccine, for increasing immune protection or for increasing vaccine efficacy in an individual to be vaccinated.
